Europäisches Patentamt

European Patent Office

Office européen des brevets

(11) Veröffentlichungsnummer: **0 485 835 A1**

## (12) EUROPÄISCHE PATENTANMELDUNG

(21) Anmeldenummer: **91118727.6**

(22) Anmeldetag: **04.11.91**

(51) Int. Cl.5: **A61L 15/07**

(30) Priorität: **14.11.90 DE 4036200**

(43) Veröffentlichungstag der Anmeldung:
**20.05.92 Patentblatt 92/21**

(84) Benannte Vertragsstaaten:
**CH DE FR GB IT LI**

(71) Anmelder: **BAYER AG**

**W-5090 Leverkusen 1 Bayerwerk(DE)**

(72) Erfinder: **von Bonin, Wulf, Dr.**
**Droste-Hülshoff-Strasse 9**
**W-5068 Odenthal(DE)**
Erfinder: **von Gizycki, Ulrich, Dr.**
**Wiembachallee 24**
**W-5090 Leverkusen 3(DE)**

(54) **Verfahren zur Herstellung von hydraulischen Bindemitteln enthaltenden Versteifungsmaterialien, insbesondere Gipsbinden.**

(57) Ein verfahrenstechnisch vorteilhaftes und umweltfreundliches Verfahren zur Herstellung von hydraulischen Bindemitteln enthaltenden Versteifungsmaterialien, beispielsweise Gipsbinden, ist dadurch gekennzeichnet, daß man ein hydraulisches Bindemittel in Pulverform mit einem reaktiven Bindemittel vermischt, diese Mischung auf ein flächiges Material aufbringt, das so beschichtete flächige Material zu Rollen aufwickelt und vor, während und/oder nach dem Aufwickeln die Abbindereaktion des reaktiven Bindemittels ablaufen läßt.

EP 0 485 835 A1

Rank Xerox (UK) Business Services
(-/2.19/2.0)

Gipsbinden sind seit langem bekannt und werden in großem Umfang für orthopädisch-medizinische Zwecke eingesetzt.

Gipsbinden werden bisher üblicherweise hergestellt, indem man dehydratisierten Gips mit einem in Methylenchlorid gelösten Bindemittel, z.B. auf der Basis modifizierter Cellulose, anteigt und diese Paste auf Bindenmaterial, z.B. Baumwollgewebe, aufrakelt. Dann wird das Methylenchlorid durch Verdampfung entfernt, wodurch der Gips durch das Bindemittel an das Bindenmaterial gebunden wird. Nunmehr kann das Gips enthaltende Bindenmaterial geschnitten und gerollt werden, ohne daß der Gips abfällt. Das in Streifen geschnittene, Gips enthaltende Bindenmaterial wird dann auf perforierte, rohrartige Wickelhülsen oder Dorne relativ locker aufgerollt. Die so entstandene Gipsbinde wird aktiviert, indem man sie in Wasser eintaucht. Hierbei dringt das Wasser von den Seiten, von der Mantelflache und über das perforierte Kernrohr in den relativ lockeren Wickel ein, der hydrophil gebundene Gips bildet einen Gipsleim, verfestigt dann die Binde nach der Applikation und ergibt einen sogenannten Gipsverband.

Dieses Herstellungsverfahren für Gipsbinden weist den Nachteil auf, daß Lösungsmittel verwendet werden müssen, insbesondere halogenierte Kohlenwasserstoffe, die aus heutiger Sicht allenfalls in geringen Mengen tolerierbar sind und deren Entfernung auch aus der Abluft sehr aufwendig und teuer ist.

Ähnlich wird bei der Herstellung von für technische Anwendungen geeigneten Versteifungsmaterialien verfahren.

Es wurde nun ein Verfahren zur Herstellung von hydraulische Bindemittei enthaltenden Versteifungsmaterialien gefunden, das dadurch gekennzeichnet ist, daß man

- ein hydraulisches Bindemittel in Pulverform mit einem reaktiven Bindemittel vermischt,
- diese Mischung auf ein flächiges Material aufbringt,
- das so beschichtete flächige Material zu Rollen aufwickelt und
- vor, während und/oder nach dem Aufwickeln die Abbindereaktion des reaktiven Bindemittels ablaufen läßt.

Als hydraulische Bindemittel kommen insbesondere die Gipssorten in Frage, die für die Gipsbindenherstellung üblich sind. Vorzugsweise handelt es sich dabei um teildehydratisierten Gips, sogenannten Stuckgips, dessen Abbindezeit weniger als 10 Minuten beträgt. Es kommen aber auch andere hydraulische Bindemittel in Frage, insbesondere zur Herstellung von Versteifungsmaterialien für technische Einsatzzwecke. Als Beispiele seien genannt: andere Gipsmodifikationen als Stuckgips, wie α-Gips und Anhydrit, sowie Portlandzement, Tonerdeschmelzzemente, Aluminiumsilikat-Schnellzemente, Sorellzemente, Zinkoxidzemente, Puzzolanzemente und sonstige mit Wasser unter Erhärtung reagierende Materialpulver mit Zementcharakter. Bei den erfindungsgemäß einzusetzenden hydraulischen Bindemitteln in Pulverform kann es sich auch um Gemische aus zwei oder mehreren Komponenten handeln. Dem hydraulischen Bindemittel können gegebenenfalls übliche Beschleuniger, z.B. lösliche Sulfate, oder Verzögerer oder rheologische Stellmittel, z.B. Proteine oder Cellulosederivate, beigefügt sein.

Als reaktive Bindemittel kommen die verschiedensten als solche bekannten reaktiven Bindemittel oder Bindemittelgemische in Frage, die in flüssiger Form auf das hydraulische Bindemittel aufgebracht werden können und dann durch Reaktion der Gemisch-Komponenten oder durch den Einfluß von Wärme, Luft, Feuchte, Belichtung und/oder durch chemische Reaktion mit einem gegebenenfalls zusätzlich einzubringenden Reaktionspartner und/oder mit einem Katalysator, Radikalbildner oder sonstigen Starter zu einer Viskositätserhöhung, z.B. infolge einer Molekulargewichtserhöhung gebracht werden können.

Die Viskositätserhöhung kann dabei, zumindest teilweise, im anschließend gebildeten Wickel erfolgen oder in diesem zu Ende gebracht werden.

Beispiele für reaktive Bindemittel sind: Ohne Zuhilfenahme von organischen Lösungsmitteln applizierbare Silikate vom Wasserglastyp, Gipse oder Zemente bindende Salze, Epoxide, Polyepoxide, Epoxid-Härter-Kombinationen, unter dem Einfluß von Luft, Ionen- oder Radikalbildnern polymerisierende Doppelbindungssysteme, z.B. mono-, di- oder polyolefinische Monomere, etwa vom Cyanacrylat-, Acrylat-, Vinylester-, Allylester- oder Allylethertyp, vom Typ der Silane, Siloxane oder Silikone (vgl. auch DE-OS 23 57 931), vom Typ der Alkydharze und reaktiven Alkydharzverdünner, vom Typ der Cyanatharze, Phenolharze, Formaldehydharze, der Methylolverbindungen, Methylolether, vom Typ der (Poly-) Isocyanate, die unter Bildung von Polyurethanen, Polyharnstoffen, Polycarbodiimiden und/oder Polyisocyanuraten zur Reaktion gebracht werden können, gegebenenfalls unter Beteiligung der im Wickel enthaltenen Feuchtigkeit.

Es kommen auch Polycarbonsäuren, Alginate, Aluminate, Cellulose- und Stärkeverbindungen in Betracht, die z.B. mit Calciumionen oder Aluminiumionen aus dem hydraulischen Bindemittel, mit dem sie in Kontakt kommen, einer Fällung oder Härtung unterliegen können.

Als reaktive Bindemittel gelangen vorzugsweise Kombinationen von einem oder mehreren Polyolen und einem oder mehreren Polyisocyanaten zum Einsatz. Kombinationen aus Polyolen und Polyisocyanaten können gegebenenfalls noch weitere Komponenten enthalten, vorzugsweise solche, die die Reaktion zwischen Polyol und Polyisocyanat verzögern oder beschleunigen. Beschleunigende Zusätze sind bevorzugt. Solche weitere Komponenten können gegebenenfalls in Mengen von 0,05 bis 3 Gew.-% (bezogen auf das Gemisch aus Polyol und Polyisocyanat) eingesetzt werden.

Als beschleunigende Zusätze kommen z.B. aminische oder metallorganische Verbindungen oder auch sonstige, in der Polyurethanchemie als katalytisch wirksam bekannte Verbindungen in Frage.

Die Komponenten des reaktiven Bindemittels können in vorgemischter Form, getrennt und gleichzeitig oder nacheinander dem pulverförmigen hydraulischen Bindemittel zugemischt werden.

Dem hydraulischen Bindemittel kann das reaktive Bindemittel in Mengen von beispielsweise 0,3 bis 50 Gew.-%, vorzugsweise 5 bis 15 Gew.-%, zugemischt werden. Hierbei ist es von Vorteil, wenn das Gemisch aus hydraulischem Bindemittel und reaktivem Bindemittel noch pulverförmigen, streubaren Charakter behält. Es kann jedoch auch eine rakelfähige pastenartige Konsistenz aufweisen.

Es ist weiterhin von Vorteil, das frisch hergestellte Gemisch aus hydraulischem und reaktivem Bindemittel unmittelbar oder innerhalb weniger Stunden nach der Herstellung auf das flächige Material aufzubringen. Vor dem Aufbringen auf das flächige Material können dem Gemisch aus hydraulischem und reaktivem Bindemittel oder einzelnen Komponenten davon gegebenenfalls weitere Zusatzstoffe, wie Netzmittel, Tenside, Fließhilfsmittel, Farbgeber und/oder Biocide, hinzugefügt werden.

Bei den bevorzugten Kombinationen aus Polyolen und Polyisocyanaten kommen als Polyole vorzugsweise die in der Polyurethanchemie technisch zur Verwendung kommenden linearen und verzweigten, vorzugsweise bei Raumtemperatur flüssigen Polyesterpolyole und Polyetherpolyole in Betracht. Von besonderem Interesse sind trifunktionelle und höherfunktionelle Typen, die durch Anlagerung von Ethylenoxid und/oder Propylenoxid an tri- und höherfunktionelle Starter, z.B. an Trimethylolpropan, Glyzerin, Pentaerythrit, Sorbit, Zucker oder Zuckergemische, Triethanolamin, Ethylendiamin, Polyethylenpolyamin, Polypropylenpolyamin, Ethanolamin und/oder Diethanolamin erhalten werden können und OH-Zahlen über 5, vorzugsweise zwischen 30 und 400, insbesondere zwischen 150 und 300, aufweisen.

Als Polyisocyanate kommen vorzugsweise ebenfalls in der Polyurethanchemie technisch zur Verwendung kommende aliphatische, araliphatische, heterocyclische und aromatische Polyisocyanate in Betracht. Vorzugsweise werden solche Polyisocyanate verwendet, deren Dampfdruck im Bereich 10 bis 50°C sehr niedrig ist. Dann ist bei deren Handhabung keine Gefährdung zu befürchten. Bei derartigen Polyisocyanaten handelt es sich vorzugsweise um bei Raumtemperatur flüssige Polyisocyanate wie polymerisiertes, trimerisiertes, biuretisiertes, allophanatisiertes oder mit geringen Mengen Wasser umgesetztes Hexamethylendiisocyanat, Isophorondiisocyanat oder solche Typen von aromatischen Mehrkern-Polyisocyanaten, wie sie auf den Wegen der Phosgenierung von Anilin-Formaldehyd-Kondensaten und deren hydrierte Formen technisch zugänglich sind. Es kommen auch andere flüssige Polyisocyanate in Betracht, auch aliphatisch-aromatische Mischtypen, z.B. solche auf Basis Isophorondiisocyanat oder Toluylendiisocyanat oder sogenannte Isocyanat-Prepolymere, d.h. Isocyanatgruppen enthaltende, vorzugsweise flüssige, oligomere Umsetzungsprodukte von Polyolen mit Polyisocyanaten.

Es ist von Vorteil, wenn die Komponenten solcher reaktiven Bindemittelmischungen aus Polyol und Polyisocyanat ineinander löslich sind. Es können auch Polyolgemische und/oder Polyisocyanatgemische zum Einsatz kommen,

Das stöchiometrische Verhältnis von OH- zu NCO-Gruppen in Polyol-Polyisocyanat-Gemischen kann in weiten Grenzen schwanken, Vorzugsweise werden mit Abweichungen von +/- 50 Gew,-% stöchiometrische Verhältnisse eingehalten. In speziellen Fällen ist es auch möglich, das Verhältnis der eingesetzten Polyisocyanate und Polyole bis auf jeweils 3 Gew,-% der stöchiometrisch äquivalenten Menge oder auch noch darunter zu reduzieren, Besonders bevorzugt ist der Einsatz von reaktiven Bindemitteln, die 90 bis 130 Gew,-% der in Bezug auf die Polyolkomponente stöchiometrisch erforderlichen Menge an Polyisocyanatkomponenten enthalten.

Diese Verhältnisse gelten auch dann, wenn man die reaktive Kombination flüssiger Bindemittelkomponenten vor, während oder nach dem Eintrag in das pulvrige hydraulische Bindemittel herstellt.

Für das Mischen mit dem hydraulischen Bindemittel sind technisch übliche Pulvermischer ohne Probleme einsetzbar, beispielsweise Schaufel-, Propeller- oder Planetenmischer.

Als flächiges Material, auf das die Bindemittelmischung aufgebracht wird, kommen die verschiedensten flexiblen, vorzugsweise textilen Substrate in Frage. Sie können beispielsweise aus Fäden, Fasern, Drähten oder Folien bestehen. Vorzugsweise handelt es sich um Vliese, Papiere, Gewirke oder Gewebe oder Mischformen davon.

Vorzugsweise werden aus Baumwolle gefertigte Bindengewebe eingesetzt, wie sie für die konventionelle Gipsbindenfertigung üblich sind. Als textile Substrate kommen aber auch solche in Betracht, die unter Verwendung von z.B. Glasfasern, Kohlenstoffasern, Polyaramidfasern, Metallfasern, Polyolefinfasern, Polyolefin-Hochmodulfasern, Polyesterfasern, Polyacrylnitrilfasern, Polyamidfasern und Fasern aus veredelter Cellulose sowie aus Fasergemischen und/oder Fadengemischen hergestellt worden sind.

Auf einen m² des flächigen Materials können z.B. 200 bis 1000 g, vorzugsweise 400 bis 800 g, insbesondere 500 bis 700 g des Gemisches aus hydraulischem und reaktivem Bindemittel aufgebracht werden.

Das flächige Material kann vor oder nach dem Aufbringen des Bindemittelgemisches oder vor oder nach dem Aufwickeln zugeschnitten werden, z.B. zu Einzelbinden.

Das Aufbringen des vorzugsweise pulvrigen oder gegebenenfalls pastenartigen Bindemittelgemisches kann im Umkehr- oder, vorzugsweise, im Direktverfahren z.B. durch Aufstreuen, Aufwalzen, Aufblasen, Aufrakeln, auf elektrostatische Weise oder nach beliebigen anderen, keine Lösungsmittel erfordernden Verfahren erfolgen.

Das Bindemittelgemisch kann auf dem flächigen Material eine homogene Fläche bilden, es kann aber auch in Form von Streifen, Punkten, durchbrochenen Flächen oder in Form von Mustern aufgebracht werden, beispielsweise um das Tränkwasser besser eindringen zu lassen oder um gezielte Versteifungseffekte zu erreichen.

Gegebenenfalls kann eine einmal aufgebrachte Schicht des Bindemittelgemisches, z.B. durch Rütteln oder durch Vibration, zur Ausbildung inhomogener Verteilungen und/oder durchlässigerer Bereiche gebracht werden. Es ist im allgemeinen vorteilhaft, die Mischung aus reaktivem und hydraulischem Bindemittel an das flächige Material leicht anzudrücken.

Es ist häufig vorteilhaft, das Abbinden des reaktiven Bindemittels mit dem hydraulischen Bindemittel durch Wärmezufuhr zu beschleunigen. So kann man z.B. während oder nach dem Aufbringen des Bindemittelgemisches auf das flächige Material z.B. durch Wärmeleitung, Strahlung, Mikrowellen, Heizgase oder eine Kombination solcher Maßnahmen Wärme zuführen oder entstehen lassen. Es ist im allgemeinen von Vorteil, die für das Aufbringen des Bindemittelgemisches eingesetzten Maschinen und Werkzeuge nichthaftend auszurüsten, z.B. durch Überzüge aus Polyolefinen, Silikonen, Perfluorpolyethylenen oder durch die Verwendung von Trennfolien.

Falls man das Abbinden des reaktiven Bindemittels durch Wärmezufuhr beschleunigen möchte kann man beispielsweise auf eine Temperatur von 30 bis 90°C, vorzugsweise von 50 bis 80°C erwärmen. Häufig ist es ausreichend, wenn diese Temperatur nur für kurze Zeit eingehalten wird, beispielsweise für 0,5 bis 60 Minuten.

Man kann das Ausreagieren des reaktiven Bindemittels vorzugsweise auch ohne zusätzliche Wärmezufuhr vor sich gehen lassen, beispielsweise indem man die frisch hergestellte Binde sofort zu einem Wickel aufrollt, wobei der rohrförmige Wickeldorn (ø z.B. 0,3 bis 5, vorzugsweise 0,8 bis 1,5 cm), wie bei Gipsbinden üblich, zweckmäßigerweise bereits gelocht ist, und die Abbindereaktion im aufgerollten Wickel ablaufen läßt. Dies kann auch in der vorgesehenen Endverpackung erfolgen. Hierbei kann das hydraulische Bindemittel im Wickel so fixiert werden, daß es aus dem Wickel bei Lagerung, Transport und Anwendung nicht oder nur in tolerierbarem Ausmaß herausrieselt.

Das reaktive Bindemittel, insbesondere bei Einsatzmengen unter 15 Gew.-%, bezogen auf das Gesamtgewicht des Versteifungsmaterials, behindert in ausgehärtetem Zustand überraschenderweise das Eindringen des Wassers in den Wickel und zu dem hydraulischen Bindemittel nicht unzumutbar und behindert auch nicht die Versteifungsreaktion des mit dem Wasser gebildeten Leims des hydraulischen Bindemittels, z.B. Gipsleims. Auch läßt sich der Bindenwickel nach dem Anfeuchten mit ausreichend Wasser ohne Probleme wie bei einer konventionell hergestellten Gipsbinde, gegebenenfalls sauberer und weniger schwierig, abwickeln und orthopädisch verwenden.

Eine spezielle Ausführungsform der Ausrüstung von Binden-Wickeln mit den erfindungsgemäß zu verwendenden Bindemittelgemischen, die ein reaktives Bindemittel enthalten und pulvrigen Charakter haben, wird durch deren Eigenschaft ermöglicht, auch bei Raumtemperatur im Laufe der Zeit auszuhärten, wobei die Rieselfähigkeit des Gemisches im Laufe der Zeit zugunsten einer haftenden Modifikation zurücktritt. Diese Ausführungsform vereinfacht die lösungsmittelfreie Herstellung von Versteifungsmaterialien bedeutend.

Diese Ausführungsform kann man z.B. realisieren, indem man während des Aufwickelns eines Bindengewebes das vorzugsweise pulvrige, gegebenenfalls auch pastenartige Bindemittelgemisch unmittelbar nach seiner Herstellung, d.h. in noch nicht abgebundenem Zustand, in den sich bildenden Wickel in definierter Menge einstreut oder einträgt. Hierzu sind z.B. Schüttelrutschen, Streuaggregate und Schlitzdüsen geeignet.

Das reaktive Bindemittel kann hierbei z.B. zwischen dem bereits gebildeten Wickel und das beim Wickeln an diesen sich anlegende, einlaufende, beschichtungsfreie Bindengewebe eingebracht werden, so daß das Gemisch aus hydraulischem und reaktivem Bindemittel lagenweise zwischen Wickelkörper und neuer Gewebelage eingequetscht wird.

Dieses gelingt deswegen ohne größere Schwierigkeiten, weil das Gemisch aus hydraulischem und reaktivem Bindemittel eine gewisse Eigenhaftung besitzt und daher durch den Gewebedruck nicht herausgepreßt, sondern zu haftenden flächigen Aggregaten verformt wird. Die Dichte und Durchlässigkeit dieser so gebildeten Schichten kann durch den Wickeldruck und die beim Wickelprozeß eingehaltenen Zugkräfte gesteuert werden.

Hierbei ist es von Vorteil, daß das erfindungsgemäß zu verwendende Bindemittelgemisch auch in nicht abgebundenem Zustand nicht mehr zum Stauben neigt. Das verbessert die Raumluftsituation bei der Bindenherstellung in besonderem Maße.

Die so hergestellten Wickel binden dann im Laufe der Zeit ab, wobei der Abbindeprozeß bis in die Endverpackung hineinverlegt werden kann Durch den Abbindeprozeß wird das Herausrieseln des in den Wickel eingebrachten hydraulischen Bindemittels zuverlässig verhindert.

Nach dem Abbindeprozeß liegt das hydraulische Bindemittel in Form eines durch das reaktive Bindemittel gebundenen, mehr oder weniger feinen Kornes vor. Überraschenderweise behindert diese Struktur nicht das Abbinden des hydraulischen Bindemittels mit Wasser, sondern sorgt im Gegenteil dafür, daß das Wasser beim Eintauchen des Wickels für z.B. 10 bis 60 Sekunden sehr schnell und gleichmäßig in diesem eindringt, wobei nur ein geringer Überschuß an Wasser, also eine begrenzte Wassermenge, vom Wickel aufgenommen wird. Weiterhin wird nur wenig hydraulisches Bindemittel mit dem überschüssigen Wasser aus dem Wickel herausgetragen. Dadurch wird gegenüber der konventionellen Herstellung von Gipsbinden eine signifikante Verbesserung der Sauberkeit bei der Handhabung solcher mit Wasser getränkter Binden erreicht. Andererseits wird durch die körnige Struktur des erfindungsgemäß gehandhabten Bindemittelgemisches die Verstreichbarkeit und die Modellierbarkeit der erfindungsgemäß hergestellten Versteifungsmaterialien nicht oder nur wenig beeinträchtigt.

Wenn man auf die erfindungsgemäße Weise Gipsbinden herstellt, kann man das Schneiden der Binden, deren Aufwicklung und Verpackung nach konventionellen Methoden der Gipsbindenherstellung vornehmen. Gleiches gilt auch für andere Formen der Anwendung erfindungsgemäß hergestellter Versteifungsmaterialien.

Erfindungsgemäß erhältliche Versteifungsmaterialien können z.B. im medizinisch-orthopädischen Bereich, zur Herstellung von Abdrücken, Masken und Formkörpern, zur Armierung von Kunststoffteilen oder als Schutzwicklung gegen mechanische und/oder thermische Einflüsse, zu Isolierungen, zu Brandschutzzwecken, zu Abdichtzwecken, in der Verbindungstechnik und für die Versteifung von Konstruktionselementen angewendet werden. Dabei können erfindungsgemäß hergestellte Versteifungsmaterialien, insbesondere Gipsbinden, auf übliche Weise aktiviert werden, indem man sie aufgerollt auf geeignete, z.B. gelochte Hülsen kurzzeitig in Wasser eintaucht. Die Eintauchzeit kann z.B. 10 bis 60 Sekunden bei einer Temperatur von 0 bis 80°C, vorzugsweise 15 bis 30°C betragen.

Das erfindungsgemäße Verfahren hat den Vorteil, daß es staubfrei aber "trocken" ist, d.h. keine Lösungsmittel benötigt werden. Auch wird keine belastende Abluft erzeugt, die nur aufwendig und kostspielig zu reinigen wäre. Das bedeutet gegenüber dem Stand der Technik eine erhebliche Vereinfachung.

Im folgenden wird das erfindungsgemäße Verfahren beispielhaft erläutert. Teile und Prozente beziehen sich auf das Gewicht, soweit nichts anderes angegeben ist.

Beispiele

In den Beispielen wurden folgende Materialien verwendet:

10 cm breite und 300 cm lange oder endlose Streifen aus Baumwollgewebe, wie es üblicherweise für die Herstellung von Gipsbinden eingesetzt wird, wobei das Metergewicht des Gewebestreifens 2,56 g betrug.

Gipspulver, wie es als sogenanntes Halbhydrat oder Stuckgips für die Gipsbindenfertigung nach konventionellen Verfahren eingesetzt wird.

Polyol A, ein technisches Anlagerungsprodukt von 80 Mol Propylenoxid und 20 Mol Ethylenoxid an Sorbit, mit einer OH-Zahl von 175.

Polyol B, ein technisches Anlagerungsprodukt von gleichen Teilen Ethylenoxid und Propylenoxid an Glycerin, mit einer OH-Zahl von 250.

Polyol C, ein technisches Anlagerungsprodukt von 60 % Ethylenoxid und 40 % Propylenoxid an Sorbit, mit einer OH-Zahl von 8.

Isocyanat A, ein technisches Biuretisierungsprodukt von Hexamethylendiisocyanat (Desmodur® N, der Bayer AG), mit einem Isocyanatgehalt von 21 %.

Isocyanat B, ein technisches Mehrkernpolyisocyanat aus der Phosgenierung von Anilin-Formaldehyd-Kondensaten, mit einem Isocyanatgehalt von 31 % (Desmodur® 44 V, der Bayer AG).

Auf einem schnell laufenden Schaufelmischer (Lödigemischer) wurden bei Raumtemperatur aus dem Gips und den Polyolen und Polyisocyanaten folgende Bindemittelgemische hergestellt, die alle rieselfähig oder streufähig waren:

Gemisch 1:

150 Teile Gipspulver wurden vorgelegt, dazugegeben wurde ein Gemisch aus 10 Teilen Polyol A und 6,9 Teilen Polyisocyanat A.

Gemisch 2:

150 Teile Gipspulver wurden vorgelegt, dazugegeben wurden zunächst 6,9 Teile Polyisocyanat A und dann 10 Teile Polyol A.

Gemisch 3:

150 Teile Gipspulver wurden vorgelegt, dazugegeben wurde ein Gemisch aus 10 Teilen Polyol A und 4,6 Teilen Polyisocyanat B.

Gemisch 4:

Vorgelegt wurden 150 Teile Gipspulver, dazugegeben wurden zunächst 10 Teile Polyol A, dann 4,6 Teile Polyisocyanat B.

Gemisch 5:

Vorgelegt wurden 100 Teile Gipspulver, dazugegeben wurden 15 Teile eines Gemisches aus 100 Teilen Polyol C und 0,2 Teilen Polyisocyanat B.

Beispiel 1

Das Baumwollgewebe wurde auf eine streifenförmige und mit einem Trennmittel auf Silikonbasis besprühte Polyethylenfolie gelegt und unter einem Streuer so hindurchgefahren, daß in getrennten Ansätzen jeweils 1 $m^2$ des Baumwollgewebes mit jeweils 600 g eines frisch hergestellten Gemisches der Typen 1 bis 5 belegt wurde.

Die jeweils aufgestreute Schicht wurde mit einer nichthaftend ausgerüsteten Walze schwach festgedrückt. Dann wurde das bestreute Baumwollgewebe durch einen Heiztunnel gefahren, wo es 1 Minute lang auf Temperaturen zwischen 60 und 80°C (ansteigend) erwärmt wurde.

Nach dem Austritt aus dem Heiztunnel wurde das nunmehr mit einer haftenden Bindemittelschicht versehene Baumwollgewebe auf 3 m abgelängt, auf einen rohrförmigen, gelochten Wickeldorn aufgewickelt und dann auf einer Verpackungsanlage in Polyethylen-kaschierte Aluminiumfolie eingeschweißt, wie es auch bei der Gipsbindenherstellung nach konventioneller Methode geschieht, wenn sie vor der Anwendung besonders geschützt werden soll.

Zur Applikation wurden die Wickel nach 2 Wochen der Verpackung entnommen. Durch das inzwischen erfolgte vollständige Abbinden der Gemische 1 bis 5 waren die Wickel ohne wesentliches Herausrieseln des Gipses handhabbar. Sie wurden 30 Sekunden lang in Wasser von 18°C eingetaucht und manuell etwas gewalkt. Dann wurde ein Prüfkörper aus Pappkarton, Durchmesser 8 cm, damit umwickelt und glattgestrichen. Nach 4 Minuten war der so hergestellte Gipsverband versteift. Härtungsverhalten und Applizierbarkeit entsprachen etwa einer auf übliche Weise hergestellten, mit 600 g Gipsmasse pro $m^2$ ausgerüsteten 10 cm-Gipsbinde, jedoch mit dem Unterschied, daß das Tränkwasser, sowie die beim Arbeiten herausgedrückte wäßrige Phase erheblich weniger den Charakter von Gipsbrühe, sondern mehr von leicht getrübtem Wasser hatte und daher ein wesentlich saubereres Arbeiten möglich wurde als bei üblichen Gipsbinden.

Beispiel 2

Es wurde gearbeitet wie in Beispiel 1, jedoch wurden dem Gipspulver jeweils noch 3 Teile entwässertes Magnesiumsulfat zugesetzt. Durch die beschleunigende Wirkung des Magnesiumsulfats härteten in der Applikationsprüfung die Wickel bereits in 2,5 Minuten aus.

Beispiel 3

Es wurde gearbeitet wie in Beispiel 1, jedoch wurden bei der Herstellung der Gemische jeweils noch 3 Gew.-% (bezogen auf das Gemisch) eines Tensids bestehend aus einem Additionsprodukt von 40 Mol Ethylenoxid an 1 Mol Abietinsäure zugesetzt, um die Wasserbenetzbarkeit zu erhöhen. Bei der Applikationsprüfung wurde nur 16 Sekunden in Wasser eingetaucht, wobei sich analoge Verarbeitungseigenschaften wie in Beispiel 1 ergaben.

Beispiel 4

Das Bindengewebe wurde auf einen Wickeldorn befestigt und zu einem Wickel gewickelt. Dabei lief der aufzuwickelnde Gewebestreifen von oben kommend nach unten in einem Winkel von 45° auf den Wickeldorn zu. Mit einer Schüttelrutsche wurden in getrennten Ansätzen die Gemische 1 bis 5 auf den sich bildenden Wickel gestreut, so daß die Pulver auf der Wickeloberfläche abgelegt und durch den Wickelprozeß zwischen frisch einlaufendem Gewebe und Wickel eingeschlossen wurden. Die Menge des Bindemittelgemisches wurde so eingestellt, daß 600 g der Bindemittelgemische 1 bis 5 pro $m^2$ Bindengewebe im Wickel abgelegt wurden.

Dann wurden die Wickel bei 3 m abgelängt und in einer Papierhülse verpackt. Nach 24 bis 60 Stunden war der Abbindeprozeß abgeschlossen und beim Öffnen der Verpackung rieselte nahezu kein Bindemittel aus dem Wickel heraus.

Die Applikationsprüfung verlief analog Beispiel 1.

Beispiel 5

Der gleiche Versuch (Beispiel 4) wurde wiederholt, wobei jedoch als hydraulisches Bindemittel ein Gemisch aus gleichen Teilen α-Gips und Portlandzement und als reaktives Bindemittel das Gemisch 1 und das Gemisch 2 verwendet wurde. Diese Bindemittelgemische härteten praktisch frei von Ausdehnung und Schrumpf aus.

Beispiel 6

Es wurde gearbeitet wie in Beispiel 4, jedoch wurde als Gewebestreifen ein 10 cm breiter Glasgewebestreifen mit einem Metergewicht von 5,9 g verwendet. In der Applikationsprüfung wurde ein Beispiel 1 analoges Verhalten festgestellt.

Beispiel 7

Es wurde wie in Beispiel 1 gearbeitet, jedoch wurde ein Gewebestreifen aus Polyestergarn verwendet (sogenanntes Raschelgewebe), das eine gute Querdehnung und ein Metergewicht von 3,35 g aufwies. In der Applikationsprüfung wurde ein Beispiel 1 analoges Verhalten festgestellt.

**Patentansprüche**

1. Verfahren zur Herstellung von hydraulische Bindemittel enthaltenden Versteifungsmaterialien, dadurch gekennzeichnet, daß man
   - ein hydraulisches Bindemittel in Pulverform mit einem reaktiven Bindemittel vermischt,
   - diese Mischung auf ein flächiges Material aufbringt,
   - das so beschichtete flächige Material zu Rollen aufwickelt und
   - vor, während und/oder nach dem Aufwickeln die Abbindereaktion des reaktiven Bindemittels ablaufen läßt.

2. Verfahren nach Anspruch 1, dadurch gekennzeichnet, daß als hydraulisches Bindemittel in Pulverform dehydratisierter Gips, $\alpha$-Gips, Anhydrit, Portlandzement, Tonerdeschmelzzement, Aluminiumsilikat-Schnellzement, Sorellzement, Zinkoxidzement, Puzzolanzement und/oder sonstige mit Wasser unter Erhärtung reagierende Materialpulver mit Zementcharakter zum Einsatz kommen.

3. Verfahren nach Ansprüchen 1 und 2, dadurch gekennzeichnet, daß als reaktive Bindemittel oder Bindemittelgemische solche zum Einsatz gelangen, die in flüssiger Form auf das hydraulische Bindemittel aufgebracht werden können und dann durch Reaktion der Gemisch-Komponenten oder durch den Einfluß von Wärme, Luft, Feuchte, Belichtung und/oder durch chemische Reaktion mit einem gegebenenfalls zusätzlich einzubringenden Reaktionspartner und/oder mit einem Katalysator, Radikalbildner oder sonstigen Starter zu einer Viskositätserhöhung gebracht werden können,

4. Verfahren nach Anspruch 3, dadurch gekennzeichnet, daß als reaktive Bindemittel Kombinationen von einem oder mehreren Polyolen und einem oder mehreren Polyisocyanaten zum Einsatz gelangen.

5. Verfahren nach Ansprüchen 1 bis 4, dadurch gekennzeichnet, daß dem hydraulischen Bindemittel 0,3 bis 50 Gew,-% reaktive Bindemittel zugemischt werden,

6. Verfahren nach Ansprüchen 1 bis 5, dadurch gekennzeichnet, daß das Gemisch aus hydraulischem Bindemittel und reaktivem Bindemittel pulverförmigen, streubaren Charakter aufweist,

7. Verfahren nach Ansprüchen 1 bis 6, dadurch gekennzeichnet, daß dem Gemisch aus hydraulischem und reaktivem Bindemittel oder einzelnen Komponenten davon Netzmittel, Tenside, Fließhilfsmittel, Farbgeber und/oder Biocide hinzugefügt werden.

8. Verfahren nach Anspruch 4, dadurch gekennzeichnet, daß das Verhältnis von OH- zu NCO-Gruppen in Polyol-Polyisocyanat-Gemischen um bis zu +/- 50 Gew.-% vom stöchiometrisch erforderlichen Verhältnis abweicht.

9. Verfahren nach Ansprüchen 1 bis 8, dadurch gekennzeichnet, daß 200 bis 1000 g des Gemisches aus hydraulischem und reaktivem Bindemittel auf einem $m^2$ des flächigen Materials aufgebracht werden.

10. Verwendung von Versteifungsmaterialien hergestellt nach einem der Ansprüche 1 bis 9 im medizinischorthopädischen Bereich, zur Herstellung von Abdrücken, Masken und Formkörpern, zur Armierung von Kunststoffteilen oder als Schutzwicklung gegen mechanische und/oder thermische Einflüsse, zu Isolierungen, zu Brandschutzzwecken, zu Abdichtzwecken, in der Verbindungstechnik und für die Versteifung von Konstruktionselementen.

Europäisches
Patentamt

# EUROPÄISCHER RECHERCHENBERICHT

Nummer der Anmeldung

EP 91 11 8727

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | US-A-3 649 319 (D.F. SMITH)<br>* Beispiel 1 *<br>--- | 1-3,5,10 | A61L15/07 |
| X | US-A-2 655 148 (J.J. EBERL)<br>* Ansprüche 1-11 *<br>--- | 1-3,5,10 | |
| A | GB-A-1 504 972 (NATIONAL RESEARCH)<br>* Seite 2, Zeile 64 - Zeile 67 *<br>* Seite 3, Zeile 25 - Zeile 35 *<br>--- | 1,2,6 | |
| A | EP-A-0 234 403 (BAYER)<br><br>----- | | |
| | | | **RECHERCHIERTE SACHGEBIETE (Int. Cl.5 )**<br><br>A61L |

Der vorliegende Recherchenbericht wurde für alle Patentansprüche erstellt

| Recherchenort | Abschlußdatum der Recherche | Prüfer |
|---|---|---|
| DEN HAAG | 25 FEBRUAR 1992 | PELTRE CHR. |